# EUROPEAN PATENT SPECIFICATION

(11) **EP 0 458 861 B1**
(45) Date of publication and mention of the grant of the patent: **03.04.1996**
(21) Application number: 90903464.7
(22) Date of filing: 26.01.1990
(51) Int. Cl.: A61K 31/44

(54) **COMPOSITION FOR REPARATION AND PREVENTION OF FIBROTIC LESIONS**
ZUSAMMENSETZUNG ZUR REPARATUR UND VORBEUGUNG FIBROTISCHER SCHÄDEN
COMPOSITION DE REPARATION ET DE PREVENTION DE LESIONS FIBROTIQUES

(30) Priority: 15.02.1989 JP 35436/89
(43) Date of publication of application: 04.12.1991
(73) Proprietor: MARGOLIN, Solomon B., Dallas, Texas 75225 (US)
(72) Inventor: MARGOLIN, Solomon B., Dallas, Texas 75225 (US)
(74) Representative: Baker, Colin John
(86) International application number: US9000449
(87) International publication number: WO9009176

(56) References cited:
- EP-A- 0 383 591
- US-A- 3 644 626
- US-A- 3 655 897
- US-A- 3 715 358
- US-A- 3 721 676
- US-A- 3 974 281
- US-A- 4 042 699
- US-A- 4 052 509
- ABSTRACTS OF THE 66TH ANNUAL MEETING OF THE FEDERATION OF AMERICAN SOCIETIES FOR EXPERIMENTAL BIOLOGY, New Orleans, Louisiana, 15th - 23rd April 1982, page 1550, abstract no. 7480; S. MARGOLIN: "Removal of interstitial pulmonary fibrosis (asbestos-induced) by oral chemotherapy with pirfenidone"

## Description

The present invention relates to the use of 5-methyl-1-phenyl-2-(1H)- pyridone for the manufacture of medicaments.

Herein, the term "anti-fibro", "anti-fibrotic" or "anti-fibrosis" refers to prevention of pathological polymerization of collagen in, for example, lung fibrosis, arteriosclerosis, prostatic hypertrophy, keloid, myocarditis, collagen disease, scar and wrinkle and reparation as well as normalization of the existing pathological fibrotic tissues.

5-Methyl-1-phenyl-2-(1H)-pyridone itself is a known compound and its pharmacological effects are disclosed, for example, in Japanese Patent Application KOKAI (Laid-Open) Nos. 87677/1974 and 1284338/1976 as an anti-inflammatory agent including antipyretic and analgesic effects. US Patents Nos. 3,839,346, issued October 1, 1974; 3,974,281, issued August 10, 1976; 4,042,699, issued August 16, 1977; and 4,052,509, issued October 4, 1977, also describe methods of manufacture of 5-methyl-1-phenyl-2-(1H)-pyridone and its use as an anti-inflammatory agent and the disclosures of those patents are incorporated herein by reference. Abstracts of the 66th Annual Meeting of the Federation of American Societies of Experimental Biology, New Orleans, Louisiana, April, 1982, p.1550, Abstract No.7480 mentions the use of 5-methyl-1-phenyl-2-(1H)-pyridone in the treatment of pulmonary fibrosis.

It has been discovered by the present inventor that the compound has other medical uses in relation to fibrotic diseases and certain respiratory diseases.

Powerful anti-inflammatory glucocorticoids (hormones relation to carbohydrate metabolism) such as hydrocortisone or prednisolone administered in very large doses have repeatedly been shown to be ineffective against fibrotic disease. These glucocorticoids do not arrest or remove such life-threatening fibrotic lesions. The glucocorticoids may be effective, however, as anti-inflammatory agents under such condition that they may temporarily ameliorate the secondary acute inflammation flare-ups which intermittently occur in tissues or organs damaged by fibrotic disease. Indeed, excessive and prolonged administration of glucocorticoids in pulmonary fibrotic disease may cause destruction of tissues, due to fibrosis or an exacerbation and acceleration of the fibrotic destruction.

Antopol (1950) was the first of many investigators who found that the anti-inflammatory glucocorticoids readily enhance fibrotic degeneration of lung tissues. Similarly, the non-steroidal anti-inflammatory agents such as aspirin, salicylates, phenylbutazone, indomethacin, various phenylacetic acid derivatives, and the like have also failed to arrest formation of, or cause repair of progressive, chronic fibrotic damage to lung tissues, prostatic tissues and musculoskeletal tissues.

Accordingly, it is a principal object of the present invention to provide compositions for the reparation and prevention of fibrotic lesional tissue.

It is an additional object of the invention to provide such compositions that comprise 5-methyl-1-phenyl-2-(1H)-pyridone as an active anti-fibrotic ingredient.

Other objects of the present invention, as well as particular features and advantages thereof, will be elucidated in, or be apparent from, the following description and the accompanying drawing figure.

The present invention is directed to the use of 5-methyl-1-phenyl-2-(1H)-pyridone for the manufacture of a medicament for the prevention of fibrotic lesions. Preferably the lesions are associated with pulmonary fibrosis.

The present invention is also directed to the use of 5-methyl-1-phenyl-2-(1H)-pyridone for the manufacture of a medicament for the arrest, repair, reparation and/or prevention of fibrotic lesions associated with: the prostrate glands including benign prostatic hypertrophy, coronary infarcts, cerebral infarcts, myocardiac fibrosis, musculoskeletal fibrosis, post-surgical adhesions, liver cirrhosis, renal fibrotic disease, fibrotic vascular disease, scleroderma, Alzheimer's disease, diabetic retinopathy, glaucoma, and skin lesions.

The present invention is further directed to the use of 5-methyl-1-phenyl-2-(1H)-pyridone for the manufacture of a medicament for the relief of chronic respiratory disease caused by: accumulation in the lungs by inhalation of polluted air or industrial dust, black lung of miners or silicosis.

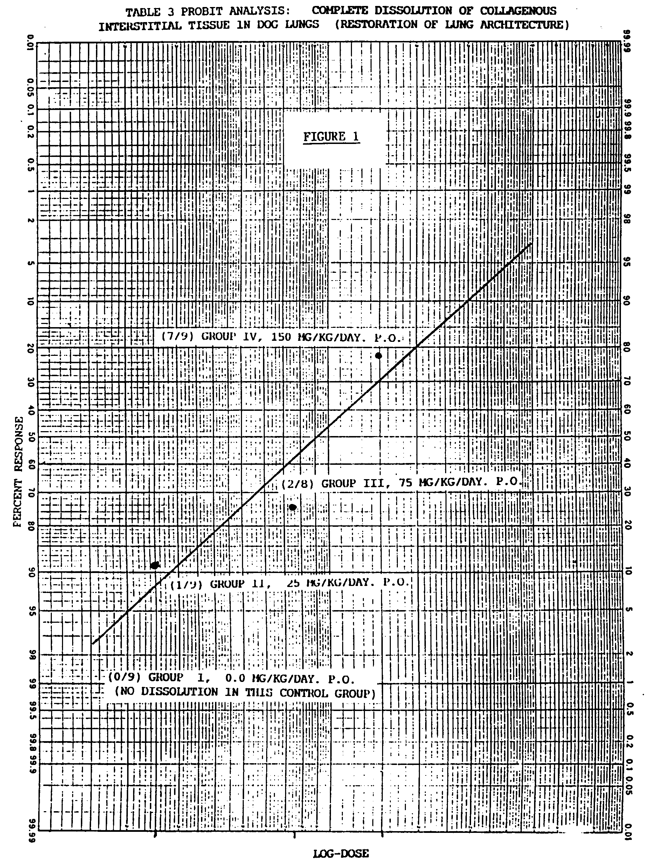

Figure 1 is a graph representing data of animal tests shown in Table 3, indicating the effect of the drug of the present invention.

The "anti-fibrotic" activity described herein differs from "fibrinolytic" or "anti-fibrin" activity. The "fibrinolytic" or "anti-fibrin" activity refers to the biological ability of a pharmaceutical substance to (1) prevent fibrin formation (prevent formation of a blood clot) or (2) lyse or dissolve a previously formed blood clot.

The "anti-fibrotic" activity discovered by the present inventor and as used herein refers to the ability of an active substance to (1) prevent an excessive pathologic accumulation of collagenous scar or connective tissue in various body structures and organs (usually triggered by some injury, allergy, infection, or by some inherited genetic aberration), or (2) cause the non-surgical removal or biological dissolution of an existing excessive and pathologic accumulation of fibrotic collagenous tissue.

### A. CONNECTIVE TISSUE PROTEINS OF MAMMALS

Three major classifications of connective tissue proteins are recognised with the largest portions consisting of collagen types (70 to 80%) and elastin types (15 to 20%). A miscellaneous group constitutes the third and smallest class.

The general biochemical characteristics of the collagen types which constitute the principal protein (1) in normal white connective tissue and (2) in scar or fibrotic tissue, are summarized in Table 1, as contrasted with elastin types. For example, collagen is sparingly soluble in water, but readily converted to water soluble gelatin upon boiling in an acid or alkali. In contrast, the highly water soluble elastin does not convert to gelatin upon boiling in an acid or alkali.

The elastin constitutes the principal protein of yellow connective tissue found in elastic structures such as the walls of larger blood vessels and walls of lung alveoli.

Investigations on the molecular biochemical level of tissues have demonstrated a very slow turnover rate for metabolic processes involving fibrotic lung collagen. In fact, the metabolic rate is measured in years. By contrast, the metabolic rates of the other connective tissue collagens including elastin and the like are measured and expressed in hours and days (White, Handler, and Smith, 1973, page 983).

### B. INTERSTITIAL PROLIFERATION (HYPERPLASIA) OF FIBROBLAST-TYPE CELLS IN LUNGS AND OTHER ORGAN TISSUES

The synthesis of various collagens found in scar or fibrotic structures takes place in fibroblast cells which then extrude the collagen into the surrounding matrix. During this wound repair process, there are (1) a rapid proliferation and increase in the number of fibroblasts at the site, and (2) a sharp rise in the rate of the synthesis and extrusion of collagen. If these two phenomena are not prevented, the pathologic and progressive accumulation of collagen would cause polymerization and fibrotic disease (for example, impairment of respiratory function, impaired circulatory function via fibrotic changes in arterial walls, fibrotic degeneration of renal and liver function, degenerative musculoskeletal function, fibrotic degeneration of cardiac muscle or skeletal muscle, fibrotic degenerative changes in neuronal tissues in the central nervous system as well as the peripheral nervous system, etc.). [S. L. Robbins, R. S. Cotrans, V. Kumar, "Pathologic Basis of Disease", 6th edition, pages 40-84, Saunders, Phildelphia, Pennsylvania (Pub.)].

With pulmonary interstitial fibrotic hyperplasia, small and firm nodules are palpable throughout the lung tissue, and upon gross examination are recognized from their opaque, airless structure to be foci of abnormal accumulations of fibrotic connective tissue. Such foci vary in size and color according to their age. Their aggressive and continued enlargement and coalescence ultimately leads to collagenous solidification of large segments of the lungs.

These enlarging foci also impinge upon the lung capillaries thereby to reduce pulmonary blood flow, and at the same time, impede lymphatic drainage from the lungs. As a consequence, exudate accumulates within the alveoli, and secondary thickening of the alveolar wall ensues. These interacting processes sharply reduce the efficiency of the gaseous exchange in the lung alveoli, which is a primary function of the normal lung.

In addition, these pulmonary fibrotic alternations and accumulations raise the pulmonary blood vessel resistance and lead to cor pulmonale (sharply elevated pulmonary blood pressure). Prolonged elevated pulmonary blood pressure almost invariably leads to congestive heart failure in addition to the cyanosis caused by inadequate pulmonary exchange of oxygen and carbon dioxide. The prognosis is poor and the incidence of severe morbidity and deaths is high.

Furthermore, the fibrosis of the lung impairs the physiological and biochemical functions of the lung that are independent of the pulmonary gas exchange (oxygen and carbon dioxide) role of the lungs cited above. These Include:
(1) filtration, degradation, and removal of the following substances:
   (a) aged leucocytes from the blood, and
   (b) particulate matter (for example, tissue cell debris, blood cell aggregates, inert foreign matter, small thrombi); and
(2) synthesis of adequate supplies of heparin.

Heparin is a useful substance that normally prevents the formation of life-threatening blood clots in the major blood vessels (for example, cerebral and coronary blood vessels).

### C DIFFERENTIATION BETWEEN ANTI-FIBROTIC ACTIVITY AND ANTI-INFLAMMATORY ACTIVITY

Pharmacological anti-fibrotic activity as exemplified by the arrest and removal of lung scarring (interstitial hyperplasia and fibrotic foci), or pathologic fibrotic lesions in other organs and tissues described herein, is clearly distinct from and independent of any pharmacological anti-inflammatory activity.

The debilitating pathologic degeneration of organs and tissues affected by fibrotic disease continues for extended periods of time, measured in months or years, beyond the short-term (hours and days) of exacerbating inflammatory flare-ups (classical clinical and histopathological signs of edema, local heat, and leucocytic infiltration have disappeared).

The composition of this invention is effective for treatment of disease caused by the pathologic and excessive fibrotic accumulations such as benign prostate hypertrophy, coronary infarcts, cerebral infarcts, myocardiac fibrosis, musculoskeletal fibrosis, post-surgical adhesions, liver cirrhosis, real fibrotic disease, fibrotic vascular disease (atherosclerosis, varix, or varicose veins), scleroderma, Alzheimer's disease, diabetic retinopathy, glaucoma and skin lesions. The composition of this invention is also effective for the prevention of fibrotic lesions associated with pulmonary fibrosis. The pulmonary fibrosis may have been chemically induced, for example, by the anti-cancer drugs bleomycin or cyclophosphamide or by the weed killer paraquat. 5-Methyl-1-phenyl-2(1H)-pyridone (5MP2P) not only arrests the formation of new fibrotic tissue but causes removal of previously formed fibrotic collagen-containing tissue.

5MP2P can also medicinally remove pathologic collagenous tissue in fibrotic lungs and as a result eliminates or prevents:
(1) the mechanical compression or occlusion (stenosis) of blood vessels (for example, pulmonary arteries, veins, and capillaries), pulmonary bronchioles, and alveoli;
(2) the inhibition of the primary respiratory function of the alveoli of the lungs, namely, the exchange of oxygen and carbon dioxide gases; and
(3) the increased pulmonary blood vessel resistance (cor pulmonale) which readily causes fatal congestive heart failure because of the excessive workload on cardiac muscle that is engendered by the cor pulmonale.

The dramatic pulmonary anti-fibrotic activity by 5MP2P has been observed and demonstrated in laboratory animal experiments (rats, hamsters, dogs) and in humans. The anti-fibrotic activity in cardiac infarctions, benign prostatic hypertrophy, and post-operated adhesions has been observed in humans. The renal anti-fibrotic activity has been demonstrated in hamsters. In every instance, the anti-fibrotic activity was clearly distinct from any anti-inflammatory properties.

The acute toxicity of the ingredient in the medical compositions of the present invention which exerts the anti-fibrotic activity is as shown in the table below:

| ACUTE TOXICITY (LD₅₀ : mg/kg) | | | | |
|---|---|---|---|---|
| Animal | Route for Administration | | | |
| | | | 10% Ointment | |
| | p.o. (number) | i.v. (number) | i.p. (number) | p.o. (number) |
| Mouse: | 997.7(40) | 285±5(50) | 600±43(60) | 11,500± 1,100(43) |

| Rat; | | | | |
|---|---|---|---|---|
| Male: | 1,295(25) | | 430±29(42) | 12,500(10) |
| Female: | 2,300(30) | | | |
| Guinea Pig: | 810±25(30) | | 460±28(25) | |
| Rabbit: | | 280±32(12) | | |
| Cat: | 500(17) | 40(4) | | |
| Dog: | 300(11) | 200(6) | | |
| Monkey: | | 100(3) | | |

Hereafter the present invention is described by referring to examples below.

The anti-fibrotic activity measured against pulmonary fibrosis was found to be quite dissimilar to and independent of anti-flammatory activity when these activities were assayed in rats and hamsters. Experiments in dog and human clinical trials affirm these findings. 5MP2P has been extensively studied for oral anti-fibrotic activity in laboratory animals and in humans. The anti-fibrotic effect in pulmonary fibrosis was demonstrated upon oral administration:
(1) in diets or by gavage to rat or hamsters,
(2) oral capsules in dogs, and
(3) oral administration to humans.

### Reference EXAMPLE 1

The results of a histopathological examination of the lungs of rats for fibrosis (interstitial hyperplasia) after receiving 300mg/kg body weight of 5MP2P in the diet for three months are summarized in Table 2. The individual microscopic readings of the lung are also shown in Table 2, where a score schedule of 0, 1, 2, and 3 reflects the degree of fibrosis.

The data in Table 2 reveal a statistically significant reduction in the amount of fibrosis in rats receiving 5MP2P as compared to placebo control rats (Group 1). The mean score for the controls (Group 1) was 1.63 ± 0.23, and for Group IV (5MP2P, 300mg/kg body weight daily was 0.95 ± 0.23.

Student's T value was 2.43, with P less than 0.02 (highly significant statistically).

In male and female Beagle dogs, the anti-fibrotic activity was found to be a direct function of the dosage of 5MP2P administered, a classical pharmacological dose-response (Table 3, Figure 1). Lung tissues examined microscopically, and scored on a schedule of 0, 1, 2, and 3 for fibrosis resulted in clear demonstration of statistically significant reduction in pulmonary fibrosis in dogs given the drug as compared to control animals.

The mean score for Group I (Control) was 2.11 ± 0.31, and for Group IV, which received 5MP2P, 150 mg/kg per day orally in capsules, was 0.22 ± 0.19.

In hamsters, pulmonary fibrosis induced with crysotile asbestos was removed following oral 5MP2P (Table 4).

This anti-fibrotic activity was not simply a palliative (relieving) effect.

The asbestos-induced fibrosis did not recur after the 5MP2P had been discontinued for two months.

A similar experience has been observed in trials on human patients with pulmonary fibrosis caused by asbestos.

The results show that 5MP2P makes possible a pulmonary resolution process whereby a life-threatening solidified fibrotic lung disease can be restored to a relatively normal tissue where the alveoli are not longer collapsed or occluded. That is, the microscopic examination reveals that the tissues are regenerated and become normal, spongy lungs.

The role of 5MP2P in the therapeutic repair of fibrotic lung tissue featuring removal of fibrotic lesions, and concomitant regeneration of normal lung tissue has been observed in experimental asbestosis by histopathological examination of lung tissue specimens under the light microscope, and electron microscopy (Table 4).

Very little, if any, fibrotic alterations are seen after treatment with adequate doses of 5MP2P.

It was further demonstrated under the electron microscope that the lung cell-imbedded asbestos fibers which had initiated and maintained the extensive fibrotic lesions also had been removed. This was subsequently confirmed by ashing of lung specimens in a laboratory oven, and then determining the asbestos content.

This "clearing" property of 5MP2P affords a therapeutic pharmacological remedy for chronic respiratory disease caused by the inhalation and accumulation in the lungs of harmful foreign matter from polluted air, asbestos, industrial dust (such as grain, lime, fertilizers, cotton fibers, glass fibers, plastics and coal), resulting in asbestosis, silicosis, and/or black lung of miners, for example.

**TABLE 1**

| CONTRAST BETWEEN PROPERTIES OF COLLAGEN AND ELASTIN | | |
|---|---|---|
| Property | Collagen | Elastin |
| 1. Water soluble | - | + |
| 2. Converts to gelatin on boiling | + | - |
| 3. Primarily in white connective tissue | + | - |
| 4. Primarily in yellow connective tissue | - | + |
| 5. Primarily associated with highly elastic structure (e.g., blood vessels) | - | + |
| 6. Primarily in organ structural tissue; fibrotic or scar tissue (e.g., lung fibrosis, etc.) | + | - |
| 7. Metabolic turnover rate | low | high |

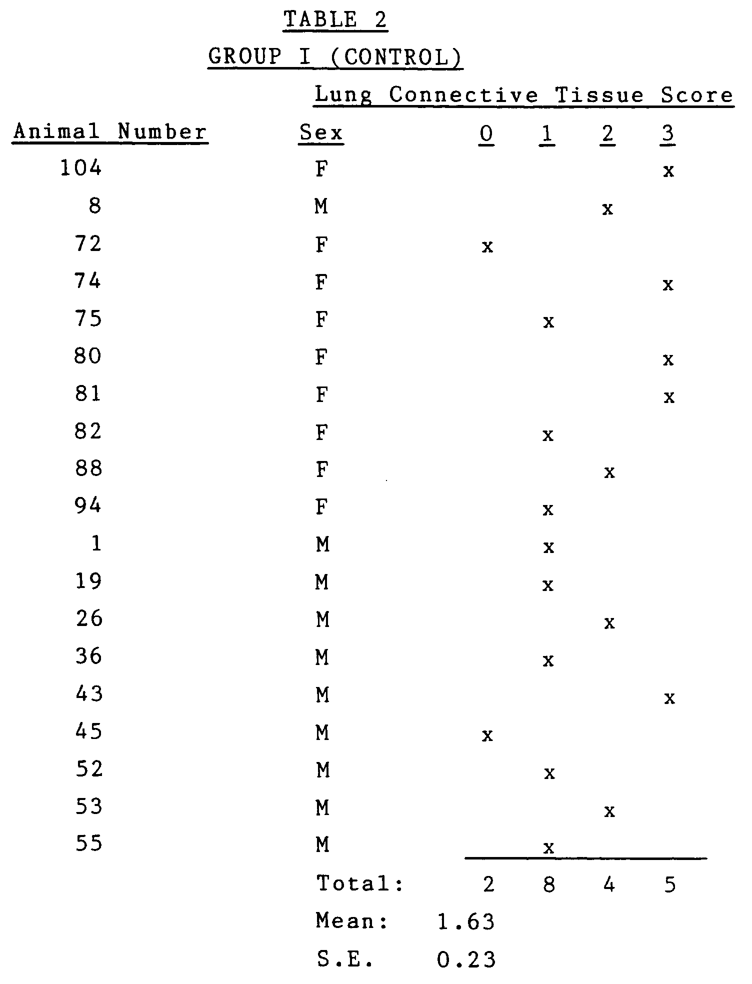

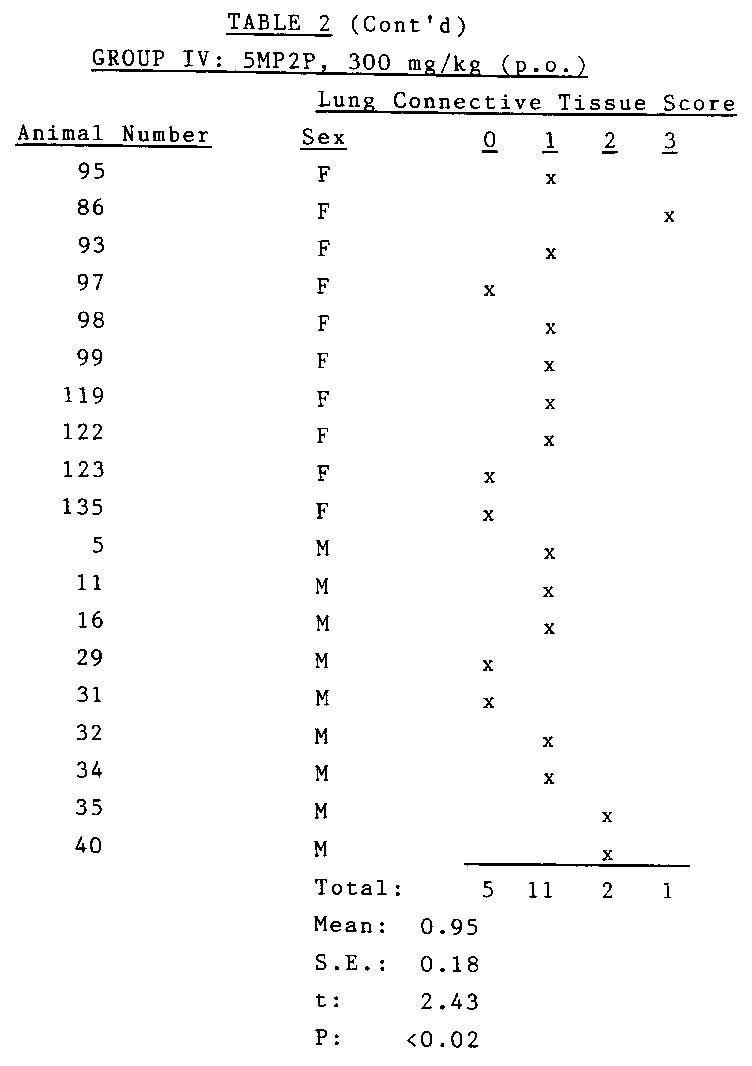

**TABLE 3**

| EFFECT OF ORAL 5MP2P UPON PULMONARY INTERSTITIAL HYPERPLASIA (FIBROSIS) IN DOGS | | | | | | | |
|---|---|---|---|---|---|---|---|
| Group | Number of Dogs | Hyperplasia Scores* | | | | Average Scores | Incidence of Normal Lung |
| | | 0 | 1 | 2 | 3 | | |
| I. Control (0.0%) | 9 | 0 | 3 | 2 | 4 | 2.11±0.31 | 0/9 |
| II. 5MP2P (16.7%) 25 mg/kg/day | 6 | 1 | 1 | 4 | 0 | 1.50±0.34 | 1/6 |
| III. 5MP2P (25.0%) 75 mg/kg/day | 8 | 2 | 2 | 3 | 1 | 1.38±0.38 | 2/8 |
| IV. 5MP2P 150 mg/kg/day | 9 | 7 | 2 | 0 | 0 | 0.22±0.15** | 7/9 |

| | | | | | | | |
|---|---|---|---|---|---|---|---|
| * Degree of Hyperplasia (fibrosis) 0 = normal tissue 1 = minimal 2 = moderate 3 = severe | | | | | | | |
| ** Highly Statistically Significant (P<0.001) | | | | | | | |

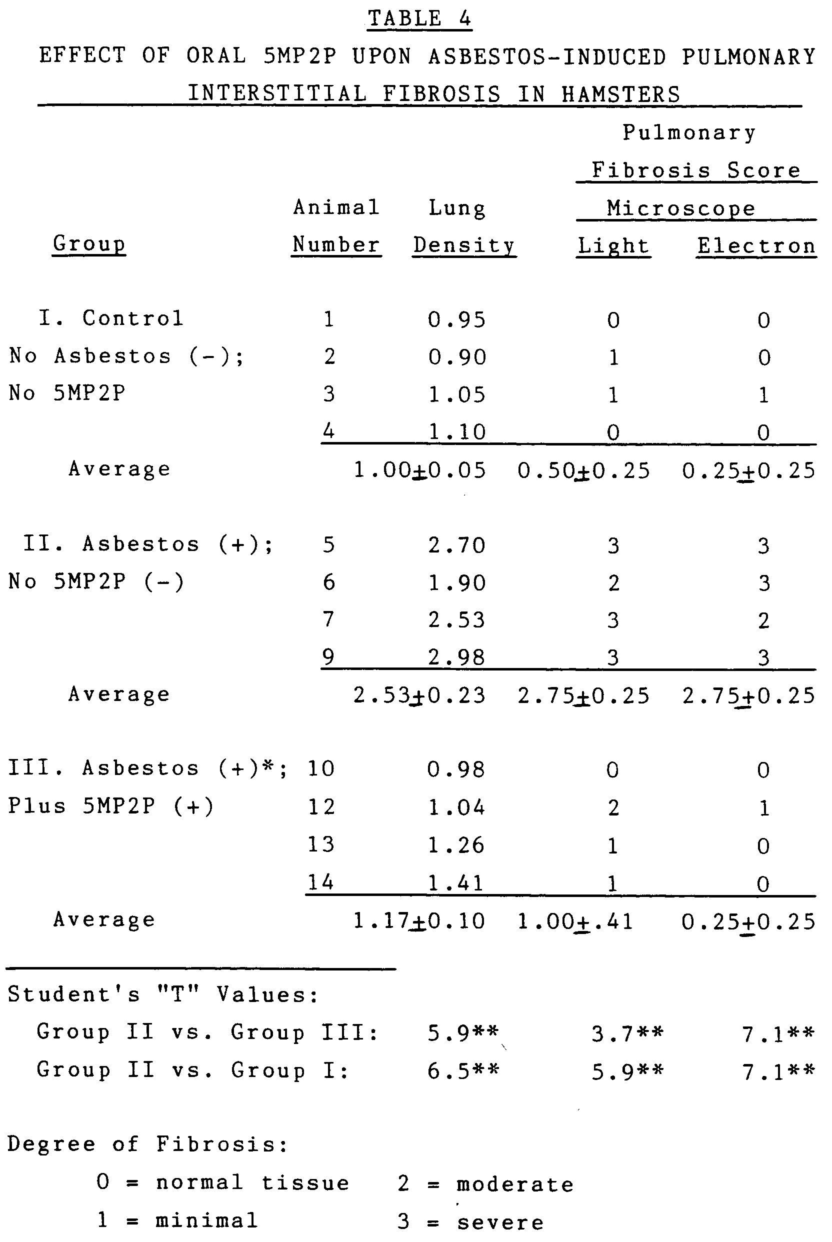

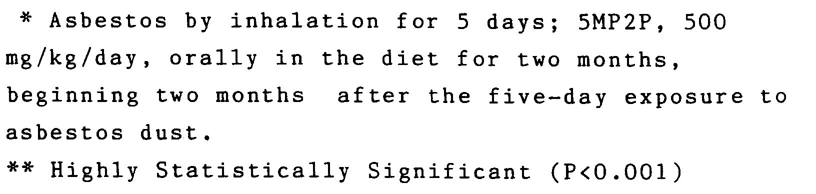

Clinical human open trials have been undertaken as follows:
1. Pulmonary fibrosis diagnosed as caused by asbestos was treated with 5MP2P and closely and objectively followed in two subjects. Clinical impressions were dramatic and highly favorable.
2. Pulmonary fibrosis diagnosed as idiopathic in nature was treated with 5MP2P and closely and objectively followed in one subject for over two years. Clinical impressions were highly favorable.
3. Benign prostate hypertrophy in three elderly subjects (66-100 years) was treated with 5MP2P with very good to excellent clinical results. Two subjects suffered from frequency, severe nocturia, incontinence, constant urgency, and in the third these symptoms were less severe. Clinically, all had enlarged prostates that explained the symptoms. The results were dramatic in the eldest subject within two weeks of therapy. Nocturia of 6-7 trips (every 60-90 minutes) per night was reduced to 1 or 2 nightly (4-5 hours apart). In the other two patients, nocturia 3-4 times (every 2-3 hours) was reduced to once nightly 4-5 hours after retiring. In all cases digital examination of the prostate revealed a detectable reduction in the size of the prostate in 3-4 weeks.
4. Fibrosis of the ventricular myocardium, an outcome of repeated coronary infarcts was treated with 5MP2P in one subject (diagnosed as terminal), with objective evidence of the reduction of the fibrosis (electrocardiogram maps and nuclear resonance determinations). The subject lived for an additional three years, despite the fact that the administration of the drug was terminated after 18 months, due to a limited supply.
5. Inhibition of excessive scar formation by direct application of 5MP2P ointment to skin lesions in 10 cases. Mild to moderate skin laceration or lesions failed to generate skin scars, or caused only minimal scarring when 5MP2P ointment was directly applied to the lesion.

Examples of medical preparations include: (1) capsules, (2) tablets, (3) powders, (4) granules, (5) syrups, (6) injection (intravenous, intramuscular, or drip administration), (7) cream, (8) ointment, (9) inhalation, (10) eye drop, (11) suppositories, (12) and pills.

The above preparations are available. Among them, capsules, injections, cream, and ointments are preferred preparations.

### TEST EXAMPLE 1

In one capsule, 100 mg, 200 mg, or 400 mg of 5MP2P is contained.

### TEST EXAMPLE 2

Hydrophilic ointment containing 5 to 10% 5MP2P.

5MP2P is usually administered to humans orally in a dose of 100 to 800 mg/time in America. It is considered to be appropriate in a daily dose of 400 to 2400mg in total for three administrations per day.

While the invention has been described in detail and with reference to specific embodiments thereof, such have been provided for purposes of illustrating the invention and are not intended as limitations thereon.

## Claims

1. Use of 5-methyl-1-phenyl-2-(1H)-pyridone for the manufacture of a medicament for the prevention of fibrotic lesions.

2. Use according to claim 1 wherein the lesions are associated with pulmonary fibrosis.

3. Use of 5-methyl-1-phenyl-2-(1H)-pyridone for the manufacture of a medicament for the arrest, repair, reparation and/or prevention of fibrotic lesions associated with: the prostrate glands including benign prostatic hypertrophy, coronary infarcts, cerebral infarcts, myocardiac fibrosis, musculoskeletal fibrosis, post-surgical adhesions, liver cirrhosis, renal fibrotic disease, fibrotic vascular disease, scleroderma, Alzheimer's disease, diabetic retinopathy, glaucoma, and skin lesions.

4. Use of 5-methyl-1-phenyl-2-(1H)-pyridone for the manufacture of a medicament for the relief of chronic respiratory disease caused by: accumulation in the lungs by inhalation of polluted air or industrial dust, black lung of miners or silicosis.

5. Use according to any one of the preceding claims wherein the medicament contains 100 to 800mg of 5-methyl-1-phenyl-2-(1H)-pyridone.

6. Use according to claim 5 wherein the 5-methyl-1-phenyl-2-(1H)-pyridone is present in a capsule in an amount of from 100 to 400 mg.

7. Use according to any one of claims 3, 4 or 5 wherein the 5-methyl-1-phenyl-2-(1H)-pyridone is present in a hydrophilic ointment in an amount of from 5 to 10%.

## Patentansprüche

1. Verwendung von 5-Methyl-1-Phenyl-2-(1H)-Pyridon zur Herstellung eines Medikaments zur Verhinderung fibrotischer Schäden.

2. Verwendung nach Anspruch 1, wobei die Schäden zusammenhängen mit bzw. auftreten bei Lungenfibrose (Bindegewebevermehrung).

3. Die Verwendung von 5-Methyl-1-Phenyl-2-(1H)-Pyridon zur Herstellung eines Medikamentes zum Arretieren, Reparieren, Wiederherstellen und/oder zur Verhinderung fibrotischer Schäden (Bindegewebevermehrungen) bei: Prostatadrüsen, einschließlich gutartiger Prostata-Hypertrophien, Herzinfarkt, Hirn-Infarkt bzw. Nerven-Infarkt, Muskel-Fibrosen, Skelettmuskel-Fibrosen, nach-chirurgischer Adhäsionen, Leber-Zirrhosen, fibrotischer Nierenerkrankung, fibrotischer Gefäßerkrankung, Hautverhärtung, Alzheimer Krankheit, Schädigung der Bindehaut bei Diabethes, Glaukom, Hautablösungen.

4. Verwendung voll 5-Methyl-1-Phenyl-2-(1H)-Pyridon zur Herstellung eines Medikaments zur Erleichterung chronischer Atmungs-Erkrankung durch: Ansammlungen in den Lungen durch Inhalation verunreinigter Luft oder industrieller Dämpfe, Staublunge der Berkläute oder Silikose.

5. Die Verwendung gemäß einem der vorhergehenden Ansprüche, wobei das Medikament 100 bis 800 mg von 5-Methyl-1-Phenyl-2-(1H)-Pyridon enthält.

6. Die Verwendung gemäß Anspruch 5, wobei das 5-Methyl-1-Phenyl-2-(1H)-Pyridon in einer Kapsel enthalten ist in einer Menge von 100 bis 400 mg.

7. Die Verwendung entsprechend einem der Ansprüche 3, 4 oder 5, wobei das 5-Methyl-1-Phenyl-2-(1H)-Pyridon enthälten ist in einer hydrophilen Salbe in einer Menge von 5 bis 10 %.

## Revendications

1. Utilisation de 5-méthyl-1-phényl-2-(1H)-pyridone pour la fabrication d'un médicament destiné à la prévention de lésions fibrotiques.

2. Utilisation selon la revendication 1, dans laquelle les lésions sont associées à une fibrose pulmonaire.

3. Utilisation de 5-méthyl-1-phényl-2-(1H)-pyridone pour la fabrication d'un médicament destiné à l'arrêt, réparation, remise en état et/ou prévention de lésions fibrotiques associées : aux glandes prostatiques y compris l'hypertrophie prostatique bénigne, les infarctus coronariens, les infarctus cérébraux, la fibrose myocardiaque, la fibrose musculo-squelettique, les adhérences post-chirurgicales, la cirrhose du foie, les affections fibrotiques rénales, les affections vasculaires fibrotiques, la sclérodermie, la maladie d'Alzheimer, la rétinophatie diabétique, le glaucome, et les lésions cutanées.

4. Utilisation de 5-méthyl-1-phényl-2-(1H)-pyridone pour la fabrication d'un médicament destiné à soulager les infections respiratoires chroniques provoquées par : accumulation dans les poumons par inhalation d'air pollué ou de poussière industrielle, pneumoconiose du mineur ou silicose.

5. Utilisation selon l'une quelconque des revendications précédentes, dans laquelle le médicament contient de 100 à 800 mg de 5-méthyl-1-phényl-2-(1H)-pyridone.

6. Utilisation selon la revendication 5, dans laquelle la 5-méthyl-1-phényl-2-(1H)-pyridone est présente dans une gélule dans une quantité de 100 à 400 mg.

7. Utilisation selon l'une quelconque des revendications 3, 4 ou 5, dans laquelle la 5-méthyl-1-phényl-2-(1H)-pyridone est présente dans un onguent hydrophile dans une quantité de 5 à 10 %.
